# FASCICULE DE BREVET EUROPEEN

(11) **EP 2 310 826 B1**
(45) Date de publication et mention de la délivrance du brevet: **06.09.2017**
(21) Numéro de dépôt: 09740383.6
(22) Date de dépôt: 24.07.2009
(51) Int. Cl.: G01N 1/40, C12M 1/26, C12M 1/00, B01L 3/00

(54) **DISPOSITIF POUR LA CAPTURE DE PARTICULES BIOLOGIQUES ET UTILISATION**
BIOPARTIKELEINFANGVORRICHTUNG UND VERWENDUNG DAVON
BIOPARTICLE CAPTURE DEVICE, AND USE THEREOF

(30) Priorité: 29.07.2008 FR 0855210
(43) Date de publication de la demande: 20.04.2011
(73) Titulaire: KABCYT, 92160 Antony (FR)
(72) Inventeur: KARKOUCHE, Bastien, 92160 Antony (FR)
(74) Mandataire: Nony
(86) Numéro de dépôt international: PCT/FR2009/051497
(87) Numéro de publication internationale: WO 2010/012941

(56) Documents cités:
- WO-A2-02/48681
- WO-A2-2008/076623
- DE-A1- 3 924 862
- DE-U1- 29 919 827
- DE-U1-202007 017 400
- FR-A1- 2 869 413
- US-A- 4 553 553
- US-A1- 2007 284 300

## Description

### Domaine de l'invention

La présente invention se rapporte au domaine de l'analyse de préparations cellulaires à des fins de diagnostic médical.

### Etat de la technique

Dans le domaine du diagnostic médical, et plus spécifiquement du diagnostic des cancers, il existe de nombreuses techniques et de nombreux dispositifs destinés à préparer des échantillons biologiques pour la réalisation ultérieure d'analyses cytologiques.

Le nombre d'actes de diagnostic médical par analyse cytologique s'est considérablement accru avec l'intérêt croissant des diagnostics cytologiques préventifs ou précoces périodiques, dont l'importance a été clairement démontrée pour l'organisation d'une prise en charge thérapeutique précoce des patients afin d'accroitre très significativement les chances de survie à long terme ou les chances de guérison.

La réalisation de diagnostics cytologiques à intervalles réguliers dans le temps est d'autant plus importante que ces techniques permettent la détection de maladies associées à un pronostic vital, le plus souvent la détection de cancers, y compris des cancers du sein, des cancers de la voie excréto-urinaire et des cancers de l'utérus.

Afin d'obtenir rapidement les résultats des tests histologiques ou cytologiques à réaliser sur les nombreux échantillons biologiques reçus quotidiennement par les anatomo-pathologistes, il a été mis au point divers systèmes intégrés permettant le traitement automatisé des échantillons biologiques.

On connait notamment des systèmes automatisés d'analyse d'images permettant, à partir d'une préparation cytologique fixée et colorée sur une lame porte-objets, d'aider le technicien à repérer les cellules ou groupes de cellules les plus pertinents pour établir un diagnostic médical.

Egalement, en amont de l'étape de lecture des préparations cytologiques, il a aussi été mis au point divers systèmes automatisés de traitement des échantillons biologiques permettant de fournir, à partir de l'échantillon biologique de départ, une préparation cytologique, prête pour l'analyse. On peut citer notamment les systèmes de ce type commercialisés par la Société Cytyc (Marlborough, MA, Etats-Unis).

De tels systèmes automatisés adaptés au traitement d'échantillons de cellules à analyser, en suspension dans un milieu liquide, sont décrits par exemple dans la demande PCT n° WO 2008/076623, ou encore dans la demande PCT n° WO 03/091704. Ces systèmes comprennent un filtre à travers lequel tout ou partie du milieu liquide est aspiré, ce qui entraine les cellules qui sont ensuite retenues par le filtre. Les cellules retenues sur le filtre sont ensuite récupérées et utilisées pour des tests cytologiques, selon des techniques appropriées.

Dans un système du type de celui décrit dans la demande PCT n° WO 2008/076623, l'aspiration du milieu liquide contenant les cellules à analyser est réalisée par une mise en dépression du compartiment situé en aval du filtre, à l'aide d'une chambre à vide. Toutefois, afin de réaliser ultérieurement une analyse cytologique fiable, il est nécessaire de retenir sur le filtre un nombre de cellules suffisant afin d'obtenir un échantillon cellulaire qui est statistiquement représentatif de la population cellulaire antérieurement prélevée. Egalement, il est important d'éviter de retenir sur le filtre un nombre de cellules trop élevé, ce qui conduirait à l'obtention d'un échantillon cellulaire dans lequel les cellules forment des amas et/ou des empilements, c'est-à-dire un échantillon à partir duquel l'analyse cytologique ultérieure serait pratiquement impossible à réaliser. Notamment, lorsque des amas ou des empilements cellulaires sont retenus sur le filtre, il existe un risque significatif que des cellules d'intérêt soient cachées dans une couche cellulaire inaccessible à l'analyse cytologique.

Afin de remédier aux inconvénients décrits ci-dessus, le dispositif décrit dans la demande PCT n° WO 2008/076623 prévoit un système de régulation de la force de la dépression générée afin d'aspirer une quantité appropriée de cellules sur le filtre. Dans ce système de régulation, la quantité de cellules retenues sur le filtre est évaluée indirectement en temps réel, par un moyen de mesure de la vitesse du flux d'air entre le filtre et la source de vide.

Dans la pratique, les systèmes automatisés d'obtention de préparations cellulaires pour analyse cytologique fonctionnent de manière satisfaisante. Toutefois, les divers dispositifs de régulation électronique contenus dans ces systèmes sont très complexes, ce qui accroit considérablement les risques de dysfonctionnement ou même d'arrêt complet du système défaillant. De plus, ces systèmes automatisés très complexes sont très onéreux, tant au moment de l'achat qu'en raison de la nécessité de procéder à des réglages et à des entretiens réguliers par des techniciens spécialisés.

Par ailleurs, il était connu un dispositif permettant de détecter des bactéries dans le sang circulant, à travers un matériau particulaire absorbant contenu dans un tube pourvu d'un piston (US 4,553,553).

Enfin, il était connu un dispositif adapté pour déposer des cellules initialement contenue dans une suspension sur un support solide (WO 02/48681). Ce dispositif inclue une première chambre contenant la suspension liquide, la chambre étant divisée en deux ou plusieurs zones contiguës. Le dispositif inclue aussi une seconde chambre destinée à recevoir l'excès de suspension lidquide et un canal à travers lequel l'excès de suspension liquide peut être déplacé depuis la première chambre vers la deuxième chambre. Le dispositif inclue également un dispositif apte à se déplacer dans la première chambre, qui comprend un corps, un élément poreux et un élément capable d'absorber un liquide.

Il existe donc un besoin dans l'état de la technique pour des systèmes alternatifs aux systèmes d'analyse cytologique existants, qui permettent l'obtention de préparations cytologiques d'une qualité au moins équivalente à celle fournie par les systèmes connus et qui possèdent une structure plus simple..

### Résumé de l'invention

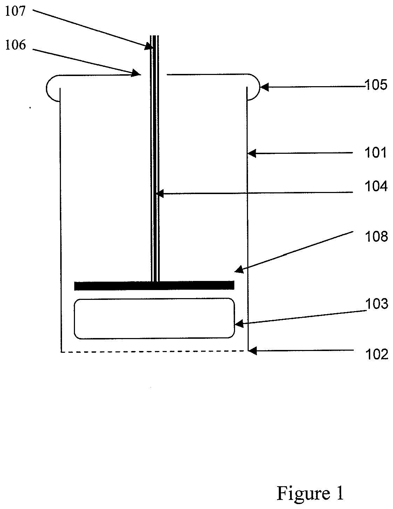

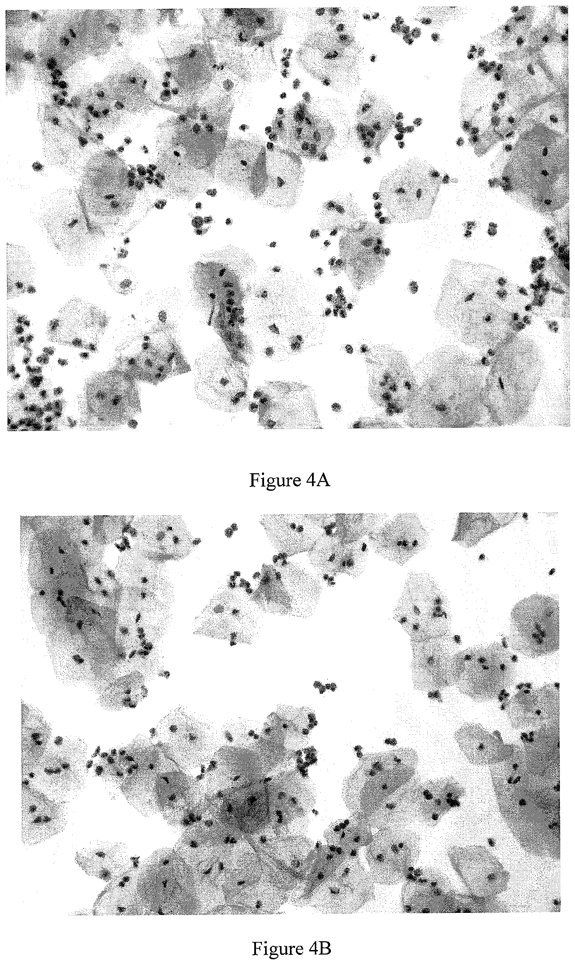

En référence aux figures 1 et 4, la présente invention a pour objet un dispositif adapté à la capture des particules biologiques en suspension dans un milieu liquide et adapté à la préparation des échantillons biologiques pour analyse cytologique, comprenant :
- un tube (101) comprenant une première et une seconde extrémités,
- la première extrémité dudit tube étant fermée par la surface d'une membrane filtrante (102) immobilisée par collage sur la section des parois dudit tube,
- un piston (104) comprenant une tige (107) reliée à un moyen d'appui (108), ladite tige coulissant selon un axe parallèle à la paroi du tube (101), ledit piston se déplaçant librement selon l'axe vertical du tube (101), et les bords du moyen d'appui (108) du piston (104) n'étant pas en contact permanent avec la surface de la paroi interne du tube (101),et
- un bloc (103) de matériau absorbant hydrophile placé à l'intérieur du tube (101), intercalé entre (i) la surface interne de la membrane filtrante (102) et (ii) le moyen d'appui (108) du piston (104), ledit matériau absorbant hydrophile ayant la propriété de gonfler lorsqu'il est mis en contact avec un milieu liquide aqueux.

L'invention concerne aussi un procédé adapté à la capture des particules biologiques en suspension dans un milieu liquide, dans lequel on met en oeuvre le dispositif décrit ci-dessus.

Cette invention a aussi trait à un procédé adapté à la réalisation d'une préparation cytologique à partir d'un milieu liquide contenant des particules biologiques en suspension, dans lequel on met en oeuvre le dispositif décrit ci-dessus.

### Rescription des Usures

La **figure 1** est un schéma représentant une section transversale verticale selon l'axe de symétrie d'un mode de réalisation du dispositif pour capturer les particules biologiques, avant utilisation.
La **Figure 2** est un schéma représentant une section transversale verticale selon l'axe de symétrie d'un mode de réalisation du dispositif pour capturer les particules biologiques, lorsque le dispositif a été plongé, sans être totalement immergé, dans un récipient contenant la suspension de particules biologiques à traiter pendant une durée suffisante pour retenir les particules biologiques à la surface de la membrane filtrante.
La **Figure 3** est un schéma représentant une section transversale verticale selon l'axe de symétrie d'un mode de réalisation du dispositif pour capturer les particules biologiques, après rétention des particules biologiques sur la membrane filtrante, lorsque la tige du piston est actionnée pour fournir une pression sur le bloc d'agent absorbant, afin de générer un flux de liquide vers l'extérieur du dispositif dans le but de détacher les particules biologiques de la membrane filtrante. Sur la Figure 3, les flèches représentent le sens d'actionnement du piston.
La **Figure 4** représente des clichés de microscopie photonique d'une préparation cytologique transférée sur une lame porte-objet, obtenue à partir d'un échantillon biologique obtenu par prélèvement de cytologie cervicale. La préparation cytologique a été fixée en milieu liquide de type PRESERVCYT®, puis soumise à une étape de coloration selon la technique de PARANICOLAOU.
La Figure 4A illustre une préparation cytologique préparée avec le dispositif de l'invention.
La figure 4B illustre une préparation cytologique obtenue avec un système automatisé à chambre d'aspiration par mise sous vide. Les échantillons présentés sur les figures 4A et 4B proviennent du même prélèvement de cytologie cervicale.
La **Figure 5** est un schéma représentant une section transversale verticale selon l'axe de symétrie d'un mode de réalisation du dispositif pour capturer les particules biologiques, immédiatement après l'immersion dudit dispositif dans un récipient contenant le fluide biologique à analyser.
La Figure 6 est un schéma représentant une section transversale verticale selon l'axe de symétrie d'un mode de réalisation du dispositif pour capturer les particules biologiques, lorsque le dispositif a été plongé, sans être totalement immergé, dans un récipient contenant la suspension de particules biologiques à traiter, pendant un durée suffisante pour retenir les particules biologiques à la surface de la membrane filtrante.

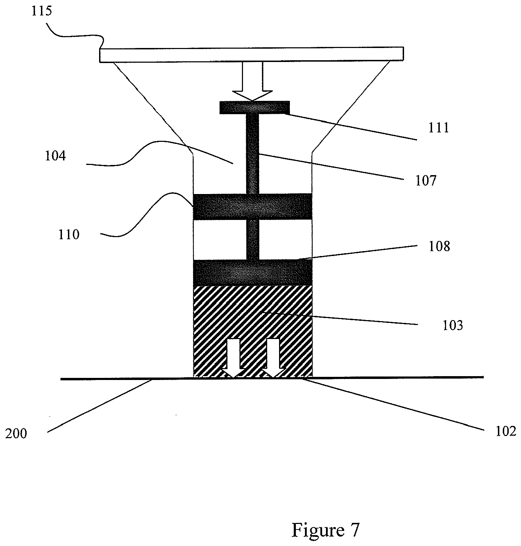

La Figure 7 est un schéma représentant une section transversale verticale selon l'axe de symétrie d'un mode de réalisation du dispositif pour capturer les particules biologiques, après rétention des particules biologiques sur la membrane filtrante, lorsque la tige du piston est actionnée pour fournir une pression sur le bloc d'agent absorbant, afin de générer un flux de liquide vers l'extérieur du dispositif dans le but de détacher les particules biologiques de la membrane filtrante. Dans le mode particulier de mise en oeuvre représenté sur la Figure 7, les particules biologiques préalablement adsorbées sur la surface de la membrane filtrante sont transférées de la membrane filtrante vers la surface d'un support d'analyse cytologique, par exemple vers la surface d'une lame porte-objet. Sur la Figure 7, les flèches représentent le sens d'actionnement du piston.
La Figure 8 est un schéma représentant un mode de réalisation particulier de la tige (107).
La Figure 9 est un schéma représentant une vue de la partie supérieure d'un mode de réalisation du tube (101) dont la géométrie est spécialement adaptée pour recevoir le mode de réalisation de la tige (107) représenté sur la Figure 8.
La Figure 10 est un schéma représentant une vue partielle d'une plateforme multi-essais selon une section transversale verticale selon l'axe de symétrie, immédiatement après l'immersion des dispositifs inclus dans ladite plateforme dans une pluralité de récipients contenant un fluide biologique à analyser.

### Description détaillée de l'invention

Le demandeur s'est attaché à mettre au point un nouveau dispositif pour capturer des particules biologiques en suspension dans un milieu liquide, essentiellement à des fins d'obtention d'échantillons biologiques pour analyse cytologique.

En particulier, le demandeur a recherché la mise au point d'un nouveau dispositif du type ci-dessus, qui soit moins onéreux que les dispositifs connus et qui permette simultanément l'obtention d'échantillons biologiques de qualité au moins équivalente à celle des échantillons biologiques préparés avec les dispositifs connus.

Au cours de ses recherches, le demandeur a montré qu'il était possible d'obtenir des échantillons biologiques de très haute qualité, notamment en vue d'une analyse cytologique ultérieure, avec un dispositif à membrane filtrante dans lequel un flux de liquide passant à travers le filtre est généré par l'absorption dudit liquide par un agent absorbant hydrophile localisé immédiatement en aval de la membrane filtrante, dans le sens du flux du liquide. En particulier, le demandeur a montré qu'avec un agent absorbant hydrophile, d'un type possédant une capacité d'absorption adaptée, on génère un flux de liquide ayant une force ou débit suffisant pour entrainer les particules biologiques contenues dans un échantillon à tester vers la membrane filtrante du dispositif en position d'immobilité, en conséquence sans qu'il soit nécessaire d'imprimer un quelconque déplacement relatif du dispositif immobile plongé dans l'échantillon à tester, par rapport audit échantillon.

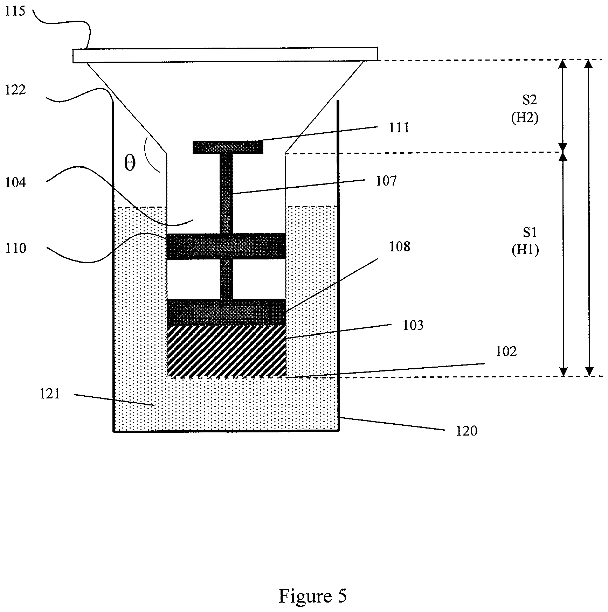

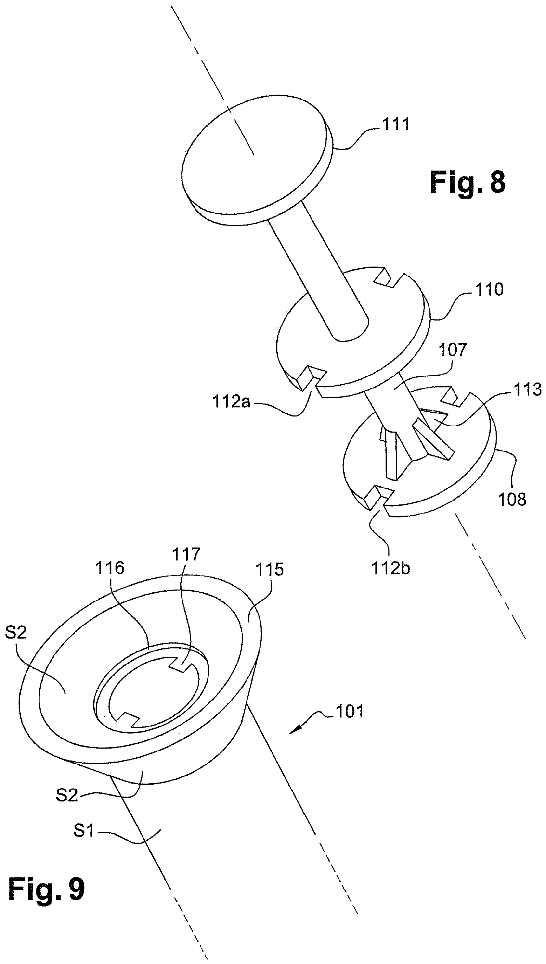

A partir de ces résultats surprenants, le demandeur a mis au point un nouveau dispositif dont un premier mode de réalisation est illustré dans les figures 1 à 4 et dont un second mode de réalisation est illustré dans les figures 5 à 7. De plus, un mode spécifique du dispositif est plus spécialement illustré dans les figures 8 et 9.

Le dispositif de l'invention pour capturer des particules biologiques en suspension dans un milieu liquide est décrit en premier lieu ci-dessous en référence aux schémas des figures 1 et 5.

La présente description a pour objet un dispositif pour capturer des particules biologiques en suspension dans un milieu liquide comprenant :
- un tube (101) comprenant une première et une seconde extrémité,
- la première extrémité dudit tube étant fermée par la surface d'une membrane filtrante (102) immobilisée par collage sur la section des parois dudit tube,
- un piston (104) comprenant une tige (107) reliée à un moyen d'appui (108), ladite tige coulissant selon un axe parallèle à la paroi du tube (101), et
- un bloc (103) de matériau absorbant hydrophile placé à l'intérieur du tube (101), intercalé entre (i) la surface interne de la membrane filtrante (102) et (ii) le moyen d'appui (108) du piston (104).

La présente invention divulgue un dispositif adapté à la capture des particules biologiques en suspension dans un milieu liquide et adapté à la préparation des échantillons biologiques pour analyse cytologique, comprenant:
- un tube (101) comprenant une première et une seconde extrémités,
- la première extrémité dudit tube étant fermée par la surface d'une membrane filtrante (102) immobilisée par collage sur la section des parois dudit tube,
- un piston (104) comprenant une tige (107) reliée à un moyen d'appui (108), ladite tige coulissant selon un axe parallèle aux parois du tube (101), ledit piston se déplaçant librement selon l'axe vertical du tube (101), et les bords du moyen d'appui (108) du piston (104) n'étant pas en contact permanent avec la surface de la paroi interne du tube (101),
- un bloc (103) de matériau absorbant hydrophile placé à l'intérieur du tube (101), intercalé entre (i) la surface interne de la membrane filtrante (102) et (ii) le moyen d'appui (108) du piston (104), ledit matériau absorbant hydrophile ayant la propriété de gonfler lorsqu'il est mis en contact avec un milieu liquide aqueux.

Par « particule biologique », on entend selon l'invention toute particule solide non soluble dans un milieu liquide aqueux qui est susceptible d'être contenue dans un matériel
biologique prélevé sur le corps d'un organisme vivant multicellulaire animal ou végétal, avantageusement un organisme vivant multicellulaire animal, de préférence un mammifère, y compris l'homme. Les particules biologiques englobent les micro-fragments tissulaires, les micro-organismes éventuels, les cellules vivantes, les cellules mortes, les corps cellulaires anucléés tels que les érythrocytes et les plaquettes (thrombocytes), les fragments, les débris cellulaires, ainsi que les cristaux éventuels et les corps étrangers solides légers. Les particules biologiques englobent donc toute substance non soluble dans un milieu liquide aqueux, y compris des substances protéiques non solubles, tels que la pectine ou des substances protéiques dérivées de la fibronectine, par exemple des substances protéiques dérivées de la fibronectine foetale qui représentent un paramètre clinique indiquant un risque d'accouchement prématuré.

Le dispositif de l'invention est décrit ci-dessous plus en détail, notamment par une description d'une variété de caractéristiques structurelles spécifiques et, le cas échéant des effets techniques générés par ces caractéristiques structurelles. Différents modes de réalisation du dispositif de l'invention sont décrits ci-dessous, en relation notamment avec l'illustration des diverses caractéristiques structurelles représentées sur les figures. Il est important de souligner qu'un mode particulier de réalisation du dispositif de l'invention peut comprendre une seule des caractéristiques techniques spécifiques qui sont détaillées ci-après, ou bien plusieurs de ces caractéristiques spécifiques en combinaison. Toutefois, simplement pour des raisons de clarté et de concision de l'exposé, les figures illustrent des modes de réalisation du dispositif de l'invention dans lesquels sont combinées plusieurs des caractéristiques techniques spécifiques détaillées ci-après, dont chacune peut être présente isolément ou en combinaison avec une ou plusieurs autres caractéristiques spécifiques, dans le dispositif de l'invention.

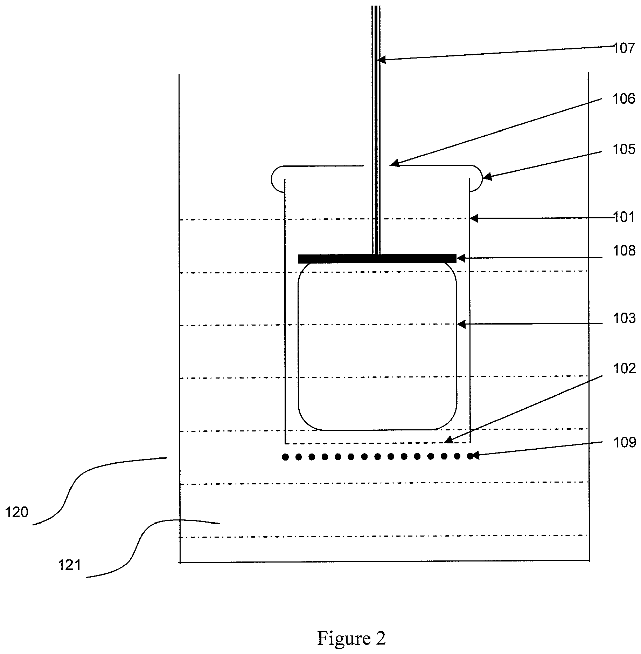

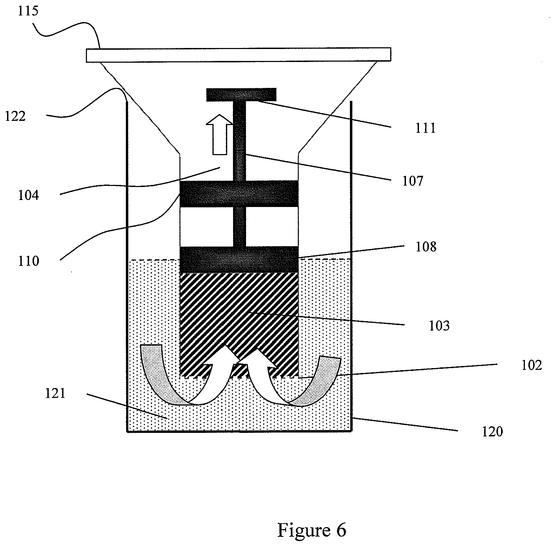

Comme cela sera décrit en détail plus loin dans la description, la capture de particules biologiques en utilisant le dispositif de l'invention est réalisée (i) en immergeant au moins l'extrémité du dispositif comprenant la membrane filtrante dans le milieu liquide contenant des particules biologiques en suspension, sans que l'extrémité supérieure du dispositif soit elle-même immergée, et (ii) en maintenant le dispositif dans ledit liquide, de préférence en position de complète immobilité au sein dudit liquide, pendant une durée suffisante pour capturer les particules sur la membrane filtrante grâce à un flux de liquide généré par l'absorption dudit liquide par le bloc d'agent absorbant. On précise que l'agent absorbant consiste en un agent absorbant hydrophile qui gonfle progressivement avec l'accroissement du volume de liquide absorbé, comme cela est représenté par exemple sur chacune des figures 2 et 6. En général, le demandeur a observé que le gonflement de l'agent absorbant se poursuit, même après que le dispositif a été retiré du liquide contenant les particules biologiques en suspension. Le demandeur pense qu'après que le dispositif de l'invention est retiré du liquide contenant les particules en suspension, la persistance du gonflement de l'agent absorbant permet l'absorption du volume réduit de liquide au contact de la membrane filtrante, en particulier au contact avec le surface externe de la membrane filtrante, ledit volume réduit de liquide étant emporté avec le dispositif du fait notamment des forces de tension superficielle et de la force du flux de liquide généré par le bloc de matériau absorbant. Le demandeur pense que le gonflement du bloc de matériau absorbant qui est observé après avoir retiré le dispositif de l'invention du milieu liquide comprenant les particules biologiques est de nature à générer une force d'aspiration résiduelle vers la lumière du tube (101), qui passe à travers la membrane filtrante (102), et qui contribue à retenir efficacement les particules biologiques sur la surface externe de ladite membrane filtrante (102), sans simultanément altérer l'intégrité physique desdites particules biologiques.

Ainsi, le bloc (103) d'agent absorbant est constitué d'un matériau hydrophile qui gonfle lorsqu'il est mis au contact d'un milieu liquide, en particulier d'un milieu liquide aqueux.

Préférentiellement, le bloc d'agent absorbant possède une capacité d'absorption d'un milieu liquide aqueux d'au moins deux fois son propre poids à l'état sec et mieux d'au moins trois ou quatre fois son propre poids à l'état sec.

Préférentiellement, le bloc d'agent absorbant possède la capacité à gonfler en augmentant de volume d'au moins deux fois par absorption d'un milieu liquide, et mieux d'au moins trois ou quatre fois, par rapport son volume initial à sec.

De manière générale, les dimensions du bloc (103) de matériau absorbant sont adaptées pour permettre une introduction aisée du bloc (103) dans le tube (101).

Les dimensions du bloc (103) sont adaptées de manière à ce que le bloc (103) puisse être déplacé aisément le long du tube (101).

Le bloc (103) de matériau absorbant est introduit à l'extrémité du tube (101) destinée à recevoir le bouchon (105) et vient se placer au contact de la membrane filtrante (102) par simple gravité. Les propriétés de gonflement du bloc (103) de matériau absorbant entrainent que la paroi externe du bloc (103) n'entre pas rapidement en contact avec la paroi interne du tube (101), mais gonfle dans le sens de son axe vertical en repoussant le piston vers le haut du tube (101), en général après 15 à 20 secondes suivant le moment de l'immersion de l'extrémité inférieure du dispositif équipée de la membrane filtrante (102).

Dans d'autres modes de réalisation du dispositif, les dimensions du bloc (103) sont telles qu'elles nécessitent une introduction et un déplacement en force du bloc (103) dans le tube (101) jusqu'à ce que le bloc (103) soit positionné à l'autre extrémité du tube (101) en contact avec la membrane filtrante (102). Le temps nécessaire après l'immersion de l'extrémité inférieure du dispositif peut varier.

Pour fabriquer un bloc (103) absorbant, l'homme du métier peut utiliser tout type d'agent absorbant hydrophile ayant les propriétés de gonflement décrites ci-dessus, qui est trouvé couramment dans le commerce.

Illustrativement, l'homme du métier peut utiliser un agent absorbant constitué de viscose, de préférence d'un matériau compressé de viscose. Dans certains modes de réalisation particuliers, on peut utiliser un matériau de viscose sous la forme d'une nappe textile « non-tissé » de viscose qui a été repliée sur elle-même un grand nombre de fois afin de former un bloc de viscose feuilleté, de dimension appropriée. De manière tout à fait préférée, ledit bloc de viscose feuilleté est soumis à une étape de compression afin d'obtenir un bloc de viscose feuilleté compressé qui possède d'excellentes propriétés absorbante du fait (i) de la grande capacité de la viscose à absorber les milieux liquides aqueux et (ii) de la force d'aspiration de liquide générée par l'augmentation de volume importante de la viscose compressée lorsqu'elle est en contact avec un milieu liquide aqueux.

Selon une autre illustration, pour l'obtention d'un bloc (103) d'agent absorbant, on peut utiliser un agent super-absorbant, bien connu par l'homme du métier.

L'homme du métier peut utiliser un agent super-absorbant de type hydrogel. Par exemple, l'homme du métier peut utiliser comme agent absorbant hydrophile un polymère du type polyacrylate de sodium réticulé, qui peut être obtenu par une réaction de polymérisation d'un acide acrylique mélangé à de l'hydroxyde de sodium en présence d'un agent initiateur de polymérisation. Les agents super-absorbants en polyacrylate de sodium réticulé sont connus en eux-mêmes et peuvent être aisément trouvés dans le commerce.

Comme agent absorbant du type agent super-absorbant, on peut aussi utiliser un copolymère de polyacrylamide, un copolymère d'anhydrique maléique et d'éthylène, de la carboxyméthyl cellulose réticulée, des copolymères d'alcool polyvinylique ou encore un oxyde de polyéthylène réticulé.

La capacité de gonflement des agents super-absorbants ci-dessus est variable mais est d'au moins 10 ou au moins 20 fois son volume initial à sec. A titre d'exemple, la capacité de gonflement d'un agent super-absorbant du type polyacrylate de sodium réticulé peut aller jusqu'à 30 à 60 fois son propre volume à l'état sec.

De manière générale, le tube (101), le bouchon (105), le piston (104) et la membrane filtrante (102) sont de types conventionnels.

Par exemple, le tube (101) équipé avec la membrane filtrante (102) peut être du type de ceux qui sont couramment utilisés comme filtres pour la mise en oeuvre des systèmes automatisés de traitement d'échantillons biologique pour l'analyse cytologique. En général, la membrane filtrante est simplement collée ou thermosoudée sur l'épaisseur de la paroi de l'une des extrémités du tube (101).

A titre illustratif, le tube (101), ainsi que la tige (107) et le moyen d'appui (108) du piston (104) peuvent être réalisés dans tout type de matière plastique, y compris en chlorure de polyvinyle (PVC), en polystyrène ou encore en polyéthylène.

De préférence, quel que soit le mode de réalisation considéré, le tube (101) se présente sous une forme monobloc, qui peut être par exemple fabriqué par moulage.

Egalement à titre illustratif, le moyen d'appui (108) du piston (104) peut être réalisé dans une autre matière, par exemple en élastomère, en latex ou en silicone.

Avantageusement, la membrane filtrante consiste en un filtre pour filtration cellulaire d'un type connu dans le domaine de la cytologie, par exemple un filtre en polyester ou en polycarbonate. A titre illustratif, on peut utiliser les membranes filtrantes appropriées commercialisées par la Société Millipore (Billerica, MA, Etats-Unis). On peut aussi citer les membranes filtrantes appropriées commercialisées par la Société Whatman-GE Healthcare (Versailles, France).

De manière générale, dans un dispositif pour capturer les particules biologiques selon l'invention, on utilise une membrane filtrante ayant une taille de pore donnée, choisie dans la gamme allant de 1 µm à 25 µm.

Dans certains modes de réalisation du dispositif pour capturer des particules biologiques selon l'invention, on utilise une membrane filtrante ayant une taille de pore donnée, choisie dans la gamme allant de 1,5 µm à 2,5 µm, préférentiellement de 2 µm. On peut par exemple utiliser la membrane filtrante référencée 7060-2511 commercialisée par la Société Whatman-GE Healthcare. Avec une membrane filtrante de ce type, le dispositif de l'invention possède la capacité à capturer la totalité des particules biologiques d'intérêt pour une analyse cytologique ultérieure, quelle que soit la nature ou l'origine tissulaire de l'échantillon biologique de départ qui a été prélevé.

Dans certains autres modes de réalisation du dispositif pour capturer des particules biologiques selon l'invention, on utilise une membrane filtrante ayant une taille de pore donnée, choisie dans la gamme allant de 3 µm à 10 µm, préférentiellement de 5 µm ou de 7µm ou de 8 µm. On peut par exemple utiliser la membrane filtrante référencée TMTP-02500 commercialisée par la société Millipore. On peut aussi utiliser la membrane filtrante référencée TTT-P02500 commercialisée par la société Millipore. On peut aussi utiliser des membranes filtrantes Cyclopore® PC commercialisées par la Société Whatman-GE Healthcare, telles que les membranes 5 µm (Refs 7060-2513 ; 7060-4713), 8 µm (Refs 7060-2514 ; 7060-4714) ou 10 µm (Refs 7060-2515; 7060-4715)Avec une membrane filtrante de ce type, le dispositif de l'invention possède la capacité à ne retenir que les cellules de grande taille, par exemple du type des cellules épithéliales contenues dans un échantillon biologique de départ obtenu après prélèvement ou frottis cervico-vaginal.

Comme cela est montré sur les figures 4A et 4B, le demandeur a montré que le dispositif de l'invention permet de capturer des particules biologiques qui sont entrainées vers la membrane filtrante (102) exclusivement par le flux de liquide généré par la force d'aspiration créée par le gonflement du bloc (103) de matériau absorbant contenu dans le dispositif en position d'immobilité, afin de réaliser ultérieurement des préparations cytologiques dont la qualité est au moins aussi bonne que celle des préparations cytologiques obtenues avec les dispositifs connus. Avec le dispositif de l'invention, on peut réaliser une grande variété de préparations cytologiques selon des techniques connues en elles-mêmes, par exemples en transférant les particules biologiques adsorbées sur la surface de la membrane filtrante dans un milieu d'analyse ou sur un support d'analyse approprié. On peut par exemple transférer les particules biologiques adsorbées sur la surface de la membrane filtrante vers un milieu liquide d'analyse, par exemple du type comprenant un agent fixateur des particules biologique, y compris un agent fixateur des cellules. Selon une autre variante classique, on peut transférer lesdites particules biologiques sur la surface d'un support pour analyse biologique, par exemple la surface d'une lame porte-objet.

Le demandeur a montré que les préparations cytologiques réalisées avec le dispositif de l'invention permettent de conserver l'intégrité physique ou biologique des particules biologiques contenues dans l'échantillon à tester. Le maintien de l'intégrité physique ou biologique des
particules biologiques dans la préparation cytologique finale est également due au fait que les particules biologiques qui sont adsorbées à la surface de la membrane filtrante (102) sont ensuite simplement transférées à la surface du support d'analyse cytologique, en général une lame de verre, en mettant en contact la surface de la membrane filtrante avec la surface du support d'analyse cytologique et en réalisant le transfert des particules biologiques de la première vers la deuxième surface en appliquant une simple pression de courte durée sur le piston, par exemple de 0,5 à 5 secondes.

Le transfert des particules biologiques de la membrane filtrante du dispositif vers le milieu d'analyse, par exemple la surface su support d'analyse cytologique, peut donc être effectué par simple contact, sans nécessiter la réalisation d'une pression de la membrane filtrante (102) sur la surface du support pour analyse cytologique. En effet, la réalisation d'une pression de la membrane filtrante (102) sur le support d'analyse cytologique, afin de transférer les particules biologique de ladite membrane filtrante vers la surface dudit support, serait de nature à générer un écrasement d'au moins une partie des particules biologiques, cette altération physique des particules biologiques étant susceptible de conduire à des préparations cytologiques finales de qualité médiocre et, dans des cas extrêmes nuire substantiellement aux résultats du diagnostic.

Comme cela a été décrit précédemment, le piston (104) du dispositif de l'invention coulisse, dans la lumière du tube (101), selon un axe parallèle à l'axe de la paroi cylindrique dudit tube (101). Dans certains modes de réalisation, le dispositif de l'invention ne comporte aucun moyen spécifique pour contraindre le coulissement du piston (104) selon l'axe prévu, du fait que l'axe de coulissement du piston (104) est déterminé comme étant perpendiculaire au plan de la surface supérieure du bloc (103) de matériau absorbant. Dans d'autres modes de réalisation, le dispositif de l'invention comprend au moins un moyen spécifique pour contraindre le coulissement du piston (104) selon l'axe prévu, comme par exemples dans les modes de réalisation du dispositif qui sont représentés respectivement dans les figures 1 et 5.

Dans le mode de réalisation illustré dans la Figure 1, la seconde extrémité du tube (101) est fermée par un bouchon (105) comprenant un orifice central (106). Dans ce mode de réalisation particulier du dispositif, la tige (107) du piston (104) coulisse, à travers l'orifice central (106), de part et d'autre de la paroi du bouchon (105). L'orifice central (106) fait fonction de guide pour la tige (107) de manière à permettre un coulissement vertical de cette dernière, selon un axe parallèle aux bords du tube (101).

Dans le mode de réalisation du dispositif de l'invention illustré dans les figures 5 et 8, la tige (107) du piston (104) coulisse verticalement, selon un axe parallèle à l'axe des parois du tube (101) grâce à la présence d'un disque (110) fixé sur la tige (107). Ce mode de réalisation particulier du dispositif sera décrit plus en détail plus loin dans la présente description.

La géométrie du dispositif selon l'invention, et en particulier la géométrie de la section transversale horizontale du tube (101), peut-être très variée.

Ainsi, dans certains modes de réalisation préférés du dispositif de l'invention, comme représenté sur la Figure 1, le tube (101) possède une section transversale horizontale circulaire auquel cas le tube (101) est de forme cylindrique. Dans ce mode de réalisation particulier, le bloc (103) de matériau absorbant possède également de préférence une forme cylindrique. De manière tout à fait préférée, le diamètre du bloc (103) de matériau absorbant est légèrement inférieur au diamètre de la paroi interne du tube (101), de manière à ce que la paroi externe du bloc (103) d'agent absorbant ne soit pas en contact avec la paroi interne du tube (101), après l'immersion de l'extrémité inférieure du dispositif dans le récipient contenant la suspension de particules biologiques.

De manière générale, le bloc (103) de matériau absorbant est maintenu en place à l'extrémité du tube (101) équipé de la membrane filtrante (102) sans nécessiter de moyen de fixation spécifique. Dans les modes de réalisation dans lesquels les dimensions du bloc (103) de matériau absorbant sont plus petites que celles de la surface interne de la paroi du tube (101), le bloc (103) est maintenu en place par simple gravité. Le maintien en place du bloc (103) est encore amélioré du fait du poids du piston (104) dont le moyen d'appui (108) peut être initialement positionné en contact avec la surface supérieure du bloc (103). Le maintien en place du bloc (103) peut être aussi assuré par pression manuelle ou mécanique légère directe sur la tige (107). Dans les modes de réalisation dans lesquels les dimensions du bloc (103) sont identiques ou supérieures à celle de la surface interne de la paroi du tube (101), le bloc (103) est maintenu en place du fait de la combinaison de la force de gravité et des forces d'appui de la paroi du bloc (103) sur la surface interne de la paroi du tube (101).

Dans d'autres modes de réalisation du dispositif de l'invention, la section transversale horizontale du tube (101) peut être de forme ovale, carrée, rectangulaire ou autre. Il va de soi que, pour des raisons simplement pratiques de fabrication et d'utilisation, le mode de réalisation préférée du dispositif de l'invention est celui dans lequel la section du tube (101) est circulaire, le tube (101) étant en conséquence de forme cylindrique. Comme on le verra dans la suite de la description, un tube (101) de forme cylindrique englobe des modes de réalisation dans lesquels le tube (101) est strictement cylindrique sur une partie seulement de sa hauteur, ledit tube (101) pouvant être de forme composite et comprendre également, en plus d'une section cylindrique, également au moins une section tronconique. On notera qu'un tube (101) possédant, comme celui représenté à la Figure 5, une section cylindrique surmontée d'une section tronconique possède, sur toute sa hauteur, et quelle que soit la section considérée, une section transversale horizontale circulaire.

Selon un autre aspect du dispositif de l'invention, les dimensions du moyen d'appui (108) du piston (104) sont choisies de manière à ce que le piston (104) se déplace librement selon l'axe vertical du tube (101). Ainsi, dans les modes de réalisation préférés du dispositif, les bords du moyen d'appui (108) du piston (104) ne sont pas en contact permanent avec la surface de la paroi interne du tube (101). Cette caractéristique particulière du dispositif de l'invention signifie
qu'un flux de gaz ou de liquide peut circuler librement, entre (i) le compartiment inférieur du tube (101) délimité par la membrane filtrante (102) et la surface inférieure du moyen d'appui (108) du piston (104), (ii) le compartiment supérieur du tube (101) délimité par la surface supérieur du moyen d'appui (108) du piston (104) et l'extrémité supérieure du tube (101) localisée au niveau du bouchon (105) et (iii) l'atmosphère extérieure avec laquelle le volume intérieur est en communication par l'orifice central (106) du bouchon (105).

A titre illustratif, lorsque le tube (101) consiste en un tube cylindrique, le moyen d'appui (108) du piston (104) est avantageusement circulaire et son diamètre est légèrement inférieur au diamètre de la paroi interne du tube (101), de manière à permettre un déplacement aisé du piston le long de l'axe vertical du tube (101). Par exemple, la présente invention englobe le mode de réalisation dans lequel le diamètre de la paroi intérieure du tube (101) est de 21 mm et le diamètre du moyen d'appui (108) du piston (104) est de 20 mm.

Un autre mode de réalisation particulier du dispositif de l'invention pour capturer des particules biologiques en suspension dans un milieu liquide est illustré, à différentes étapes d'un procédé pour sa mise en oeuvre, dans les figures 5 à 7.

En référence plus particulièrement à la Figure 5, ce mode particulier de réalisation du dispositif de l'invention comprend une partie supérieure évasée en forme d'entonnoir, qui favorise sa stabilité au sein du liquide dans lequel ledit dispositif est plongé lorsqu'il est utilisé pour une analyse, par exemple une analyse cytologique d'un prélèvement de tissu biologique.

Dans le mode de réalisation représenté dans la Figure 5, le tube (101) comprend deux sections formant une surface externe continue, respectivement :
- une première section S1 de type cylindrique dont une extrémité consiste en la première extrémité dudit tube qui est fermée par la surface d'une membrane filtrante (102), et dont l'autre extrémité forme une surface externe continue avec une deuxième section, et
- une deuxième section S2 de type tronconique, dont l'extrémité de plus petit diamètre forme une surface externe continue avec ladite première section cylindrique, et dont l'extrémité de plus grand diamètre consiste en la seconde extrémité du tube (101).

Sur la Figure 5, le dispositif de l'invention a été placé dans un récipient (120) contenant un liquide (121) à analyser. Comme on le voit sur la Figure 5, la surface externe de la section (S2) tronconique du tube (101) vient en appui, du fait de la force de gravité, sur les bords (122) du récipient (120), de manière à bloquer la translation verticale du tube (101) à une position donnée au sein du récipient (120).

Il est donc nécessaire que le diamètre externe D1 de la deuxième extrémité, c'est-à-dire l'extrémité supérieure, du tube (101) soit supérieure au diamètre interne D2 du récipient contenant le liquide à analyser.

En général, les récipients adaptés pour les analyses de prélèvements biologiques, en particulier les récipients adaptés pour les analyses de cytologie, possèdent des dimensions standard déterminées. En conséquence, les dimensions du dispositif de l'invention, et en particulier celles de la section S2 tronconique ainsi que celles de la hauteur totale du tube (101) peuvent être déterminées à l'avance pour être adaptées à chacun des types de récipients pour analyse biologique couramment utilisés.

Comme cela est représenté sur la Figure 5, une combinaison adéquate de (i) la hauteur H1 de la section S1 du tube (101), (ii) l'angle 0 formé entre les parois externes des sections S1 et S2 et (iii) la hauteur H2 de la section S2, permet que la surface de la membrane filtrante (102) localisée à la première extrémité du tube (101) soit disposée à une distance appropriée du fond du récipient d'analyse (120), de manière à ce que la surface externe de la membrane filtrante (102) :
- ne soit ni en appui, ni en contact quelconque, avec la surface du fond du récipient d'analyse (120), et
- soit positionnée à une distance réduite de la surface du fond du récipient d'analyse (120) afin de permettre la récupération de manière optimale les particules biologiques en suspension et réduire ainsi les risques de défaut de récupération de certaines particules, par exemple des particules de densité élevée, susceptibles d'être en suspension vers le fond du récipient (120).

Dans certains modes de réalisation, la hauteur H1 de la section S1 est égale à au moins deux tiers de la hauteur H totale du tube (101).

Sur le mode de réalisation du tube (101) qui est représenté sur la Figure 5, ledit tube comprend de plus un épaulement annulaire à son extrémité supérieure.

Dans ce mode de réalisation particulier du tube (101), l'extrémité de plus grand diamètre de la section S2 du tube (101) comprend un épaulement (111) annulaire plat dont le plan est perpendiculaire aux bords de la section S1 dudit tube (101). L'épaulement (11) forme un méplat, c'est-à-dire une surface annulaire dont le plan est perpendiculaire à l'axe vertical du tube (101) et dont le diamètre interne coïncide avec le plus grand diamètre de la section S2 tronconique. De manière préférée, l'extrémité de la section S2 et l'épaulement (115) forment une surface externe continue.

Préférentiellement, dans ce mode de réalisation du tube (101), les dimensions du tube (101) sont choisies de manière que (i) le diamètre externe de l'extrémité de plus grand diamètre de la section S2 soit inférieur au diamètre interne des parois verticales du récipient (120) et que (ii) le diamètre externe de l'épaulement annulaire (115) soit supérieur au diamètre interne des parois verticales du récipient (120). Selon une telle disposition, lorsque le tube (101) est engagé dans le récipient (120) d'analyse, la surface de l'épaulement (115) est en contact avec les bords supérieurs du récipient (120) (non représenté sur la Figure 5). Dans ce mode de réalisation, la distance entre la surface externe de la membrane (102) du tube (101) et la surface du fond du récipient (120) est déterminée par la différence entre (i) la hauteur H qui est la somme des hauteurs H1 et H2 respectivement des sections S1 et S2, et (ii) la hauteur entre (ii-1) la jonction
de la paroi verticale du récipient (120) avec la surface interne du fond dudit récipient et (ii-2) les bords supérieurs des parois du récipient (120).

Sur le mode de réalisation du dispositif de l'invention qui est représenté sur la Figure 5, la tige (107) du piston (104) est munie d'un disque (110) qui est localisé à une position intermédiaire entre le moyen d'appui (108) et l'extrémité supérieure de la tige (107). Un mode de réalisation de cette forme du piston (104) est représenté en détail sur la Figure 8.

Ainsi, dans certains modes de réalisation du dispositif de l'invention pour capturer des particules biologiques, ledit dispositif est caractérisé en ce qu'un disque (110) est fixé sur la tige (107), le diamètre dudit disque (110) étant déterminé de manière à permettre le guidage de la tige (107) selon un axe parallèle aux parois du tube (101). Dans ces modes de réalisation du dispositif, le disque (110) permet un coulissement du piston (104) selon un axe qui est maintenu vertical sur la totalité de la course dudit piston.

En référence aux figures 5 et 8, certains modes de réalisation du dispositif de l'invention comportent la tige (107) du piston (104) qui est équipée d'un disque (110). Dans le mode de réalisation du piston (104) représenté sur la Figure 8, ledit piston comprend un moyen poussoir (111) destiné à transmettre une force d'appui verticale, du haut vers le bas du piston, afin de transférer les particules biologiques adsorbées sur la membrane filtrante vers le milieu ou vers le support d'analyse cytologique, ou bien encore afin d'expulser une partie du liquide susceptible d'être contenu dans le tube (101), en particulier dans le bloc d'agent absorbant.

Dans un mode de réalisation particulier du piston (104) qui est représenté sur la Figure 8, la force d'appui verticale qui est exercée par l'intermédiaire du moyen poussoir (111) est transmise de manière sensiblement uniforme sur la totalité de la surface du moyen de poussée (108), grâce à un ou plusieurs renforts (113). Chaque renfort (113) est (i) solidaire, sur un premier de ses côtés, avec la paroi de la tige (107), et est (ii) solidaire sur un deuxième côté avec la surface supérieure du moyen de poussée (108). Préférentiellement, un renfort (113) est de forme triangulaire avec l'un des trois côtés qui est fixé sur la paroi externe de la tige (107) et un autre des trois côtés qui est fixé sur la surface supérieure du moyen de poussée (108). Dans ce mode de réalisation préféré, un renfort (113) à la forme d'une équerre. De manière tout à fait préférée, la longueur du côté du renfort (113) qui est solidaire du moyen de poussée (108) est égale à au moins la moitié de la distance, mieux au moins les deux tiers de la distance, séparant (i) le bord externe du moyen de poussée (108) et (ii) la paroi de l'extrémité de la tige (107) qui est fixée sur le moyen de poussée (108).

Le piston (104), lorsqu'il est équipé de renforts (113), comprend de préférence au moins deux, et mieux au moins quatre, renforts (113). En général, le nombre de renforts (113) peut être de 2, 3, 4, 5 ou 6 renforts.

La présence des renforts (113) permet une transmission de poussée verticale sensiblement uniforme sur la totalité de la surface du bloc (103) de matériau absorbant hydrophile lors de l'utilisation du dispositif, et permet en conséquence une expulsion de liquide contenu dans le dispositif, à travers la membrane filtrante (102), avec une pression sensiblement uniforme sur toute la surface de la membrane filtrante (102). Ainsi, lors de l'utilisation de ce mode de réalisation spécifique du dispositif de l'invention, le détachement des particules biologiques susceptibles d'avoir été précédemment adsorbées à la surface de la membrane filtrante (102), pour le transfert vers le milieu ou le support d'analyse cytologique, est réalisé également de manière sensiblement uniforme, à partir de la totalité de la surface externe de ladite membrane filtrante (102), en général vers la surface du support d'analyse cytologique.

Dans le mode de réalisation du piston (104) représenté dans sur la Figure 8, le disque (110) comprend deux évidements ou encoches (112). Ce mode de réalisation du piston (104) est destiné à être utilisé avec le mode de réalisation du tube (101) qui est représenté sur la Figure 9. La Figure 9 est un schéma de l'extrémité supérieure du tube (101) de la Figure 5, qui est opposée à l'extrémité sur laquelle est fixée la membrane filtrante (102). Sur la Figure 9 est représentée l'extrémité supérieure du tube (101) qui comprend :
- l'épaulement annulaire (115),
- la section S2 tronconique dont la surface interne au tube (101) est bien visible sur la Figure 9, et dont la surface externe est presque entièrement occultée sur la figure, du fait de l'effet de la perspective,
- la face interne de la jonction entre la section S2 tronconique et la section S1 cylindrique du tube (101), laquelle jonction est matérialisée, dans ce mode de réalisation, par l'existence d'un épaulement annulaire (116) comportant une série de saillies ou ergots (117).

Dans certains modes de réalisation du dispositif, non représentés sur les figures, une série de saillies ou ergots, du type de ceux représentés sur la Figure 9, est localisée dans la partie médiane de la section S1 du tube (101). Cette série spécifique de saillies ou ergots peut avoir pour fonction de bloquer le piston (104) dans une position de hauteur intermédiaire, ce qui permet de bloquer l'expansion de volume du bloc de matériau absorbant à une hauteur désirée, et donc à un volume désiré, dans le tube (101). Le blocage de l'expansion de volume du bloc de matériau absorbant entraine l'arrêt du flux de liquide de l'échantillon vers le dispositif de l'invention et stoppe ainsi l'adsorption de particules biologiques additionnelles sur la surface de la membrane filtrante. Ces modes de réalisation particuliers du dispositif de l'invention permettent de réaliser un contrôle du nombre de particules biologiques qui sont adsorbées sur la surface de la membrane filtrante, et donc aussi un contrôle de la densité de particules biologiques adsorbées sur la surface de la membrane filtrante, à la fin de l'étape de collecte des particules biologiques. Il va de soi qu'un contrôle du nombre des particules biologiques recueillies à partir de l'échantillon, y compris un contrôle de la densité de ces particules sur la membrane filtrante, permet d'accroitre encore la qualité du spécimen qui doit être analysé.

Dans le mode de réalisation du dispositif de l'invention qui est représenté sur les figures 8 et 9, le piston (104) est introduit dans la lumière du tube (101), le cas échéant en appliquant un angle entre l'axe de la tige (107) et l'axe vertical du tube (101), afin d'engager le moyen de poussée (108) sans difficulté. Puis, après avoir engagé le moyen de poussée (108) dans la lumière du tube (101), on applique au piston (104) une translation verticale, parallèle à l'axe vertical du tube (101) et on fait coïncider (i) la ou les encoche(s) (112) du disque (110) du piston (104) avec la ou les saillies (117) correspondantes du tube (101). Puis on poursuit la translation jusqu'à l'engagement complet du piston (104) dans le tube (101), c'est-à-dire jusqu'à ce que la surface inférieure du moyen de poussée (108) soit en contact avec le bloc (103) de matériau absorbant. Ce mode de réalisation qui comprend la combinaison de (i) un piston (104) comprenant un disque (110) muni d'une ou plusieurs encoches (112) et de (ii) un tube (101) comprenant un épaulement (116) muni d'une ou plusieurs saillies (117) correspondantes, permet un engagement aisé du piston (104) dans la lumière du tube (101), et simultanément prévient un désengagement non désiré dudit piston (104). Avec ce mode réalisation particulier du dispositif, il existe une faible probabilité que, une fois que le piston (104) est engagé dans la lumière du tube (101), il y ait à nouveau une coïncidence entre la ou les encoches (112) et la ou les saillies (1 17) correspondantes qui entrainerait un désengagement dudit piston.

Dans les modes de réalisation précédemment décrits dans lesquels le dispositif de l'invention est muni d'une série de saillies ou d'ergots sur la partie médiane de la Section S1 du tube (101), la translation verticale du piston (104) provoquée par l'expansion du volume du bloc de matériau absorbant est bloquée par la mise en contact du disque (110) du piston avec lesdites saillies ou ergots.

Dans encore d'autres modes de réalisation du dispositif de l'invention, le moyen de poussée (108) comprend aussi une ou plusieurs encoches (112), en général de dimension et de positionnement identiques aux encoches (112) dont est muni le disque (110). Dans certains modes de réalisation préférés, la ou les encoche(s) (112) du disque (110) et la ou les encoche(s) du moyen de poussée (108) est (sont) chacune à l'aplomb l'une de l'autre, selon l'axe principal de la tige (107). Dans d'autres modes de réalisation préférés, la ou les encoche(s) du moyen de poussée (108) est (sont) décalée(s) l'une par rapport à l'autre, selon l'axe principal de la tige (107), ce qui réduit encore le risque de désengagement du piston (104).

Dans tous les cas, dans ces autres modes de réalisation du dispositif, l'engagement du piston (104) dans la lumière du tube (101) est aisé, sans que soit accru le risque de son désengagement dudit piston.

La présente invention a aussi pour objet un procédé pour capturer des particules biologiques en suspension dans un milieu liquide comprenant les étapes suivantes :
a) placer un dispositif pour capturer des particules biologiques tel que décrit ci-dessus dans un récipient contenant un milieu liquide dans lequel des particules biologiques sont en suspension,
b) maintenir le dispositif dans ledit récipient pendant une durée suffisante pour capturer au moins une partie des particules biologiques contenues dans le milieu liquide sur la surface externe de la membrane filtrante (102) du dispositif (101),

Le dispositif de l'invention tel qu'il se présente au début de l'étape a) du procédé ci-dessus est représenté respectivement sur chacune des figures 1 et 5.

Avantageusement, le récipient contenant la suspension de particules biologiques est d'un type connu, par exemple un flacon couramment utilisé pour le conditionnement des prélèvements de cellules ou de tissus à des fins d'analyse biologique, y compris d'analyse cytologique ou histologique.

Egalement, le milieu liquide contenant les particules biologiques en suspension est d'un type connu. Le plus souvent, lorsque l'analyse cytologique projetée consiste en une analyse de types cellulaires sur des lames porte-objet, ledit liquide consiste en un liquide aqueux tamponné contenant un agent de fixation des cellules ou des corps cellulaires en suspension. En tant qu'agent fixateur, on peut citer notamment des mélanges à base d'alcool. Par exemple, on peut utiliser l'agent fixateur à base d'alcool commercialisé sous la marque SEDFIX® par la société SURGIPATH ou celui commercialisé sous la marque PRESERVCYT® par la société Cytyc ou celui commercialisé sous la marque EASYFIX® par la société Labonord. Dans d'autres modes de réalisation, par exemple lorsque l'analyse cytologique ultérieure est réalisée à partir de cellules vivantes, ledit liquide peut consister en un milieu tampon salin, préférentiellement en un milieu de culture cellulaire approprié. Dans encore d'autres modes de réalisation, ledit liquide peut consister en un fluide corporel naturel telle que l'urine, ou encore en un fluide corporel sécrété lors de pathologies, comme une ascite, un épanchement, un kyste ou encore un écoulement.

La durée de l'étape a) est variable. Il s'agit de la durée nécessaire à déplacer le dispositif de l'invention de sa position de stockage vers sa position en contact avec la suspension de particules biologiques à traiter.

A l'étape b), il suffit en général que l'extrémité du tube (101) équipée de la membrane filtrante (102) soit en contact avec le milieu liquide et que la totalité de la surface externe de la membrane filtrante soit immergée dans ledit milieu liquide. Un flux de milieu liquide est alors généré à travers la membrane filtrante (102) vers l'intérieur du tube (101), et plus particulièrement vers le bloc (103) de matériau absorbant qui est positionné à cette extrémité du tube (101). Le flux entrant de milieu liquide est généré à la fois (i) par la force de tension superficielle résultant des caractéristiques d'énergie de surface du bloc (103) de matériau absorbant et (ii) par l'action mécanique d'aspiration du milieu liquide résultant de l'augmentation de volume progressif du matériau absorbant constitutif du bloc (103).

A l'étape b) le flux entrant de milieu liquide entraine les particules biologiques vers l'intérieur du tube (101), les particules étant, selon la nature de l'échantillon biologique de départ et selon la taille de pore de la membrane filtrante (102), totalement ou seulement partiellement retenues sur la surface externe de la membrane filtrante (102) en contact avec le milieu liquide.

La durée de l'étape b) peut être aisément adaptée par l'homme du métier, en fonction de divers critères, telles que (i) la densité finale de particules biologiques retenues sur la membrane filtrante qui est désirée, (ii) la concentration de particules biologiques en suspension dans le milieu liquide de départ et (iii) la capacité d'absorption du bloc (103) de matériau absorbant.

De manière générale, quel que soit le mode de réalisation du dispositif selon l'invention qui est utilisé, la durée de l'étape a) est d'au moins 5 secondes, durée nécessaire à la génération du flux entrant de liquide vers le volume intérieur du tube (101) entrainant la capture d'un nombre minimal suffisant de particules biologiques à la surface de la membrane filtrante (102).

On a montré selon l'invention qu'avec un dispositif tel que décrit ci-dessus qui comprend un bloc (103) de matériau absorbant en viscose compressée, la capture sur la membrane filtrante (102) d'un nombre de particules biologiques adapté à leur analyse ultérieure par cytologie est obtenue pour une durée de l'étape b) allant de 5 secondes à plusieurs minutes, selon la nature de l'échantillon biologique de départ, et en particulier selon la concentration des particules biologiques en suspension dans le milieu liquide de départ. La durée de l'étape b) peut être conditionnée par le colmatage des pores de la membrane filtrante par les particules, ce qui entraine l'arrêt presque total de l'absorption et l'obtention de couches minces de particules, sans nécessiter d'outils de mesure complexes.

A la fin de l'étape b), le dispositif se retrouve dans un état qui est illustré sur le schéma de chacune des figures 2 et 6. Sur chacune des figures 2 et 6, le dispositif selon l'invention est présenté en immersion dans le récipient contenant les particules biologiques en suspension dans un milieu liquide. Le bloc (103) de matériau absorbant a augmenté de volume, par rapport à son volume de départ à l'état sec représenté sur chacune des figures 1 et 5. Sur la surface externe de la membrane filtrante (102) de la Figure 2 sont représentées les particules biologiques (109), initialement en suspension dans le milieu liquide, et qui ont été retenues. Sur chacune des figures 2 et 6, le piston (104), dont le moyen d'appui (108) est toujours en contact avec la surface supérieure du bloc (103) de matériau absorbant, s'est déplacé en position haute sous l'effet du gonflement du matériau absorbant.

A l'issue de l'étape b) du procédé, les particules biologiques retenues sur la surface externe de la membrane filtrante (102) peuvent être récupérées et traitées selon des méthodes conventionnelles d'analyse cytologique, par exemple par transfert par réplique, par pression du piston sur le bloc (103), à partir de la membrane filtrante (102) vers la surface d'une lame porte-objets, puis éventuellement la réalisation ultérieure d'une étape de coloration de la préparation, préalable à la réalisation de l'analyse cytologique, cette analyse étant généralement effectuée à l'aide d'un appareil de microscopie photonique.

De manière surprenante, comme illustré sur la Figure 4, on a montré selon l'invention que l'utilisation du dispositif décrit dans la présente description permet la réalisation de préparations pour analyse cytologique de qualité au moins égale à celles obtenues avec les systèmes connus, y compris celles obtenues avec les systèmes automatisés décrits antérieurement dans la présente description.

Ainsi, de manière surprenante, l'analyse d'une préparation cellulaire obtenue en utilisant le dispositif de l'invention montre que l'intégrité des cellules est souvent aussi bien ou même mieux préservée que lorsque un dispositif connu est utilisé.

Sans vouloir être lié par une quelconque théorie, le demandeur pense que la meilleure intégrité des cellules qui peut être observée avec le dispositif de l'invention est due au fait que le débit du flux entrant de liquide qui est généré par le bloc (103) de matériau absorbant est plus réduit que le débit du flux entrant qui est généré par les systèmes connus, en particulier par les systèmes dans lesquels ledit flux entrant est généré par mise sous vide du compartiment localisé en aval de la membrane filtrante. En conséquence, avec le dispositif de l'invention, l'arrêt des particules par la membrane filtrante provoque une décélération plus faible des particules et simultanément une altération plus faible, voire nulle, de leur intégrité physique.

L'utilisation du dispositif selon l'invention présente d'autres avantages, notamment lorsque l'échantillon biologique de départ consiste en un prélèvement dit « hémorragique », dans lequel sont contenus de larges dépôts de fibrine. En utilisant des systèmes connus avec ce type de prélèvements, on obtient en général des préparations cytologiques difficiles à analyser sur les lames porte-objet du fait de la présence de nombreux dépôts de fibrine qui ont été entrainés avec les particules biologiques d'intérêt vers le filtre.

Au contraire, avec le dispositif de l'invention, on obtient des préparations cytologiques d'excellente qualité sur les lames porte-objet, même dans les cas où l'échantillon de départ consiste en un prélèvement hémorragique, c'est-à-dire qu'on obtient des préparations cytologiques exemptes, ou sensiblement exemptes, de dépôt de fibrine. Le demandeur pense que cet avantage additionnel du dispositif de l'invention est dû au faible débit du flux entrant dans le dispositif, qui n'entraine pas les dépôts de fibrine conjointement avec les particules biologiques d'intérêt.

L'utilisation du dispositif selon l'invention est également avantageuse au cours d'une intervention chirurgicale, par exemple au cours d'une cytoponction écho-guidée en examen extemporané. On pourra indiquer à l'opérateur si l'échantillon de liquide prélevé est satisfaisant, ce qui lui permet de répéter son geste en cas de prélèvement d'un échantillon qui ne s'avèrerait pas satisfaisant. Les cytoponctions englobent celles d'un nodule mammaire, d'une métastase hépatique, ou encore d'une tumeur d'un organe profond. Une telle utilisation du dispositif de l'invention permet de réduire le risque de gestes chirurgicaux répétés, dont l'aspect invasif provoque des traumatismes inutiles chez les patients.

Comme cela a déjà été précisé ci-dessus, le dispositif de l'invention est utilisé dans des procédés de réalisation de préparations cytologiques.

Ainsi, la présente invention est également relative à un procédé adapté à la réalisation d'une préparation cytologique à partir d'un milieu liquide contenant des particules biologiques en suspension, comprenant les étapes suivantes :
a) placer un dispositif tel que défini dans la présente description dans un récipient contenant un milieu liquide dans lequel des particules biologiques sont en suspension,
b) maintenir le dispositif dans ledit récipient pendant une durée suffisante pour capturer au moins une partie des particules biologiques contenues dans le milieu liquide sur la surface externe de la membrane filtrante (102) du dispositif,
c) retirer le dispositif dudit récipient, le cas échéant par traction sur la tige (107) du piston (104), et
d) récupérer au moins une partie des particules biologiques retenues sur la membrane filtrante du dispositif.

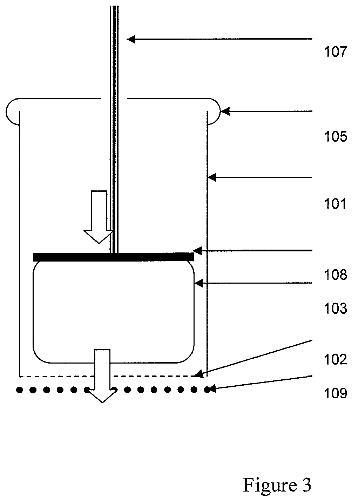

Dans des modes de réalisation avantageux de l'étape d) du procédé ci-dessus d'obtention de préparations cytologiques, une pression est exercée sur le bloc (103) par action sur le piston (104), de manière à générer un flux de liquide sortant de l'intérieur du dispositif (101) vers l'extérieur, ledit flux de liquide provoquant le détachement des particules biologiques initialement retenues sur la membrane filtrante. Ce mode particulier du procédé est illustré sur chacune des figures 3 et 7.

Une fois détachées de la membrane filtrante (102), les particules biologiques d'intérêt sont récupérés puis soumis à une ou plusieurs étapes de traitement préalables à leur analyse cytologique.

En général, les particules retenues sur la membrane filtrante (102) du dispositif de l'invention sont, à l'étape d), récupérées selon une méthode classiquement utilisée par les anatomo-pathologistes, par transfert des particules biologiques par réplique de la membrane filtrante vers la surface support d'une lame porte-objets, telle que la lame porte-objets (200) qui est représentée dur la Figure 7.. La préparation biologique, en général la préparation cellulaire, qui adhère à la surface de la lame porte-objet peut ensuite être soumise à une ou plusieurs étapes de traitements préalables à son observation, par exemple une ou plusieurs étapes de coloration spécifique ou non-spécifique, y compris des étapes de coloration au May-Grümwald Giemsa, coloration dite « Papanicolaou » carmin aluné, éosine, érythrosine, coloration de schorr, fuschine basique, hémalun de Mayer, hématéine, hématoxyline, coloration au noir Soudan, mucicarmin, nigrosine, orcéine, phloxine b, Ponceau de xylidine, réactif de Schiff, rouge Congo, etc.

Comme décrit ci-dessus, dans certains modes de réalisation de l'étape d) du procédé selon l'invention pour réaliser une préparation cytologique, on transfère au moins une partie des particules biologiques de la membrane filtrante du dispositif vers la surface d'un support d'analyse cytologique, par mise en contact de ladite membrane filtrante avec la surface dudit support d'analyse cytologique.

Egalement, dans certains modes de réalisation, ledit procédé comprend l'étape additionnelle suivante :
e) réaliser une coloration des particules biologiques transférées sur la surface dudit support d'analyse cytologique.

Selon encore d'autres modes de réalisation de l'étape d) du procédé selon l'invention pour réaliser une préparation cytologique, on transfère au moins une partie des particules de la membrane filtrante du dispositif vers un conteneur approprié, par exemple un tube de culture cellulaire, pour obtenir une préparation cytologique sous la forme d'une suspension concentrée de cellules.

La suspension concentrée de cellules obtenue à la fin de l'étape d) peut être ensuite soumise à une ou plusieurs étapes de traitement ultérieures, préalables à l'analyse cytologique.

A titre illustratif, la suspension concentrée de cellules obtenues à la fin de l'étape d) peut être incubée en présence d'anticorps détectables spécifiques de marqueurs membranaires ou de marqueurs intra-cellulaires, préalablement à l'analyse cytologique qui peut être effectuée par exemple par une technique de cytométrie de flux, le cas échéant après une incubation additionnelle avec des anticorps marqués.

Egalement, la suspension concentrée de cellules obtenues à la fin de l'étape d) peut ensuite être traitée par des techniques de biologie moléculaire, par exemple par des techniques d'hybridation in situ avec des sondes nucléiques spécifiques ou par des technique d'extraction d'ARN puis de quantification du niveau d'expression d'un ou plusieurs gènes d'intérêt, ou bien encore par des techniques d'extraction d'ADN puis de détection de mutations dans la séquence d'un ou plusieurs gènes d'intérêt.

Selon encore d'autres modes de réalisation de l'étape d) du procédé selon l'invention pour réaliser une préparation cytologique, on récupère au moins une partie des particules biologiques retenues sur la membrane filtrante par grattage de ladite membrane filtrante.

Dans certains modes de réalisation, il est possible de désolidariser la membrane filtrante (102) du reste du dispositif, et de procéder à l'inclusion de l'ensemble membrane filtrante/particules biologiques retenues dans la paraffine.

Le grattage de la membrane filtrante peut être réalisé par tout dispositif adapté d'un type connu. A titre illustratif, on peut utiliser des spatules qui sont couramment employées en culture cellulaire pour mettre en suspension des cellules cultivées qui adhèrent au support de culture, ces spatules étant aussi désignées « cell scrapers ».

Ces derniers modes de réalisation du procédé de l'invention sont avantageusement mis en oeuvre lorsqu'il est utile de récupérer des micro-fragments tissulaires à des fins d'analyse. A titre illustratif, les micro-fragments ainsi récupérés peuvent ensuite être inclus dans de la paraffine, ou dans tout autre type de résine adaptée, pour la réalisation de coupes histologiques qui seront étudiées par des techniques de microscopie, le cas échéant après avoir été soumises à une ou plusieurs étapes de coloration histochimique ou de coloration immunohistochimique appropriées. Ces modes de réalisation du procédé de l'invention sont mis en oeuvre tout particulièrement pour effectuer les analyses cytologiques des particules biologiques prélevées à partir des tissus muqueux, par grattage.

Tout particulièrement, le dispositif de l'invention permet de récupérer les micro-fragments tissulaires et cellulaires aux fins de leur analyse histologique. Cet aspect du dispositif est particulièrement utile, compte tenu du développement croissant actuel des techniques de prélèvement par ponction d'organes ou encore de grattage ou brossage des tissus selon des techniques utilisant le guidage par endoscopie assistée par des systèmes d'imagerie médicale. En effet, ce type de prélèvement qui est actuellement pratiqué de manière croissante permet l'obention de matériel de prélèvement dit « mixte », aussi désigné « cyto-biopsique ». Il s'agit d'un matériel biologique composite comprenant à la fois des cellules entière et des micro-fragments tissulaires.

Dans l'objectif d'encore mieux optimiser la mise en oeuvre d'analyses cytologiques avec un dispositif pour capturer des particules biologiques tel que défini dans la présente description, il a été aussi mis au point une plateforme multi-essais comprenant une pluralité de dispositifs selon l'invention, ladite plateforme multi-essais ayant été conçue pour réaliser simultanément une pluralité de préparations cytologiques à partir d'échantillons biologiques de départ.

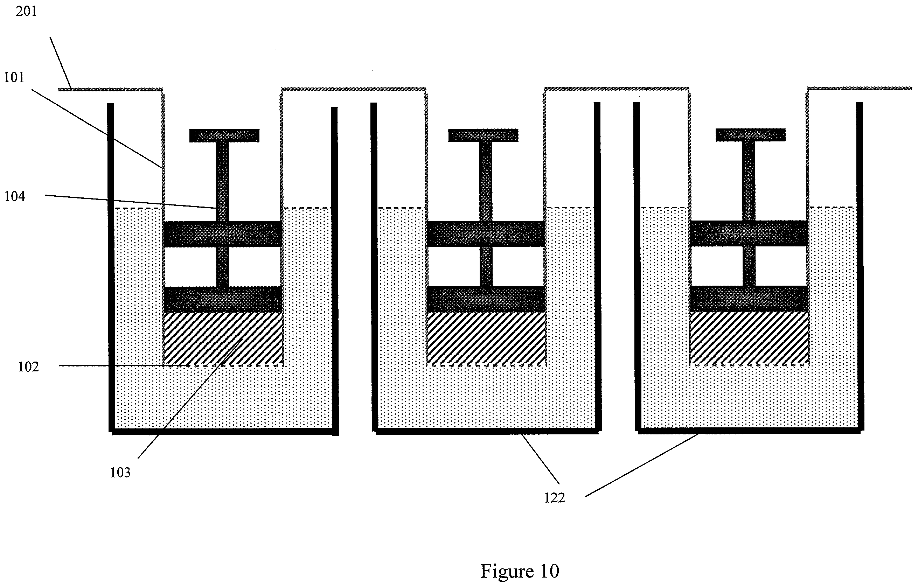

En référence à la Figure 10, qui est une coupe transversale verticale d'une vue partielle d'une plateforme multi-essais de l'invention, ladite plateforme comprend une pluralité de dispositifs pour capturer les particules biologiques qui sont ordonnés de manière déterminée sur la surface de ladite plateforme. Sur la Figure 10 est représentée une série de trois dispositifs de l'invention qui sont disposés en ligne dans la plateforme multi-essais.

Dans certains modes de réalisation d'une plateforme multi-essais de l'invention, ladite plateforme multi-essais comprend une pluralité de dispositifs pour capturer des particules biologiques qui sont ordonnés en ligne. Dans ces modes de réalisation, la plateforme multi-essais comprend avantageusement un nombre de dispositifs pour capturer des particules biologiques au moins égal à 2 et au plus égal à 100.

Au moins égal à 2 englobe au moins égal à 3, 4, 5, 6, 7, 8, 9, 10, 11, 12, 13, 14, 15, 16, 17, 18, 19, ou 20. Au plus égal à 100 englobe au plus égal à 99, 98, 97, 96, 95, 94, 93, 92, 91, 90, 85, 80, 75, 70, 65, 60, 55, 50, 45, 40, 35, 30, 25, 24, 23, 22, 21 ou 20. Dans ces modes de réalisation de la plateforme multi-essais, ladite plateforme se présente sous la forme d'un barrette comprenant une pluralité de dispositifs pour capturer des particules biologiques qui sont ordonnés entre eux selon un axe linéaire unique.

Dans certains autres modes de réalisation d'une plateforme multi-essais, la pluralité de dispositifs pour capturer des particules biologiques sont ordonnés à la fois selon une pluralité de lignes parallèles entre elles, et une pluralité de colonnes parallèles entre elles et perpendiculaires aux lignes. Chaque ligne et chaque colonne comprend une pluralité de dispositifs pour capturer des particules biologiques. Lorsque le nombre de dispositifs de l'invention dans chaque ligne est identique au nombre de dispositifs dans chaque colonne, la plateforme multi-essais peut être de forme carrée. Dans ces modes de réalisation de la plateforme multi-essais, chaque ligne ou chaque colonne comprend avantageusement un nombre de dispositifs pour capturer des particules biologiques au moins égal à 2 et au plus égal à 100.

Comme on le comprend aisément, tout autre type d'arrangement ordonné entre eux des dispositifs pour capturer des particules biologiques dans une plateforme multi-essais de l'invention est englobé par la présente invention.

Dans le mode de réalisation d'une plateforme multi-essais qui est représenté sur la Figure 10, les dispositifs pour capturer des particules biologiques sont tous inclus dans une structure de type monobloc. Dans ce mode de réalisation, la paroi d'un premier tube (101) et la paroi d'un second tube (101) situé à proximité du premier tube sont reliées entre elles par la surface supérieure de la plateforme. Dans ce mode de réalisation, (a) la paroi d'un premier tube (101), (b) la paroi d'un second tube (101) situé à proximité du premier tube et (b) la surface de la plateforme reliant les deux parois, forment une surface externe continue, ce qui matérialise la structure de type monobloc de ladite plateforme. Dans ce mode de réalisation sous forme monobloc, la structure de la plateforme multi-essais incluant les parois de chacun des tubes (101) inclus dans celle-ci, peut être réalisée par simple moulage d'un matériau polymère tel qu'un polyéthylène, un polystyrène ou un polypropylène, selon des techniques bien connues de l'homme du métier. Sur la base de chacun des tubes (101), on rapporte ensuite une membrane filtrante (102). Puis chacun des tubes (101) est ensuite équipé d'un bloc (103) de matériau absorbant, préalablement au positionnement d'un piston (104).

Dans d'autres formes de réalisation de la plateforme multi-essais de l'invention, la structure de la plateforme se présente sous la forme d'un plateau dans lequel on a pratiqué une pluralité d'évidements de manière ordonnée, chaque évidement étant destiné à recevoir un dispositif pour capturer des particules biologiques, dans sa forme de réalisation générale décrite en détail plus haut dans la présente description. Le nombre et l'arrangement spatial des évidements dans la structure de la plateforme multi-essais englobe les possibilités décrites précédemment pour l'arrangement spatial des tubes (101) inclus dans la plateforme de type monobloc.

Comme cela est représenté sur la Figure 10, chaque dispositif pour capturer des particules biologiques inclus dans la plateforme multi-essais est destiné à être introduit dans un récipient contenant un échantillon biologique à tester. Dans la pratique, il est nécessaire que l'arrangement des récipients soit compatible avec l'arrangement des dispositifs inclus dans la plateforme multi-essais. Afin de satisfaire cette contrainte technique, les récipients contenant les échantillons biologiques à tester sont avantageusement disposés au préalable dans un portoir permettant une disposition desdits récipients qui soit compatible avec l'arrangement des dispositifs au sein de la plateforme multi-essais.

L'arrangement des réceptacles pour récipients d'échantillon, dans le portoir, est « compatible » avec l'arrangement des dispositifs inclus dans la plateforme multi-essais, lorsque chacun desdits dispositifs contenus dans la plateforme peut être introduit dans chacun des récipients disposés dans ledit portoir. Bien entendu, la plateforme multi-essais peut comprendre un nombre de dispositifs pour capturer des particules biologiques qui est supérieur au nombre de récipients effectivement disposés dans le portoir. Dans cette situation, une analyse cytologique de la totalité des échantillons contenus dans lesdits récipients peut être réalisée, bien que la totalité des dispositifs contenus dans la plateforme multi-essais n'est pas utilisée.

De manière optimale, sont disposés autant de dispositifs pour capturer des particules biologiques dans la plateforme multi-essais que de récipients sur le portoir correspondant.

Par ailleurs, on utilise la plateforme multi-essais de l'invention selon les mêmes techniques que celles utilisées pour un dispositif pour capturer des particules biologiques, qui ont déjà été détaillées plus haut dans la présente description.

Ainsi, la présente invention est aussi relative à une plateforme multi-essais comprenant une pluralité de dispositifs pour capturer des particules biologiques tels que définis dans la présente description.

Préférentiellement, ladite plateforme multi-essais se présente sous la forme d'une structure monobloc.

La présente invention concerne aussi un système adapté à la capture des particules biologiques en suspension dans un milieu liquide, ledit système comprenant la combinaison de deux éléments :
- un premier élément qui consiste en une plateforme multi-essais telle que définie ci-dessus, qui comprend une pluralité de dispositifs pour capturer des particules biologiques disposés, dans ladite plateforme, selon un arrangement déterminé, et
- un portoir destiné à recevoir des récipients pour échantillons biologiques, lesdits récipients pouvant être disposés, dans ledit portoir, selon un arrangement compatible avec l'arrangement des dispositifs dans ladite plateforme multi-essais.

La présente invention a également trait à un procédé adapté à la réalisation d'une préparation cytologique à partir d'un milieu liquide contenant des particules biologiques en suspension, comprenant les étapes suivantes :
a) placer au moins une partie de la pluralité de dispositifs (101) pour capturer des particules biologiques contenus dans une plateforme multi-essais telle que définie ci-dessus dans un récipient, ou une pluralité de récipients, contenant chacun un milieu liquide dans lequel des particules biologiques sont en suspension,
b) maintenir le ou les dispositif(s) dans ledit ou lesdits récipient(s) pendant une durée suffisante pour capturer au moins une partie des particules biologiques contenues dans le milieu liquide sur la surface externe de la membrane filtrante (102) de chacun des dispositifs (101),
c) réaliser une translation de la plateforme multi-essais afin de retirer le ou les dispositif(s) dudit récipient ou desdits récipients correspondant(s), et
d) récupérer au moins une partie des particules biologiques retenues sur la membrane filtrante de chacun des dispositifs contenus dans la plateforme multi-essais ayant été placés dans un récipient.

La mise en oeuvre du procédé ci-dessus est par ailleurs identique à celle du procédé dans lequel un seul dispositif pour capturer des particules biologiques est utilisé. Les détails de cette mise en oeuvre sont donc par ailleurs décrit précédemment dans la présente description, en relation avec la procédé de mise en oeuvre d'un seul dispositif pour capturer des particules biologiques.

## Revendications

1. Dispositif adapté à la capture des particules biologiques en suspension dans un milieu liquide et adapté à la préparation des échantillons biologiques pour analyse cytologique, comprenant:
- un tube (101) comprenant une première et une seconde extrémités,
- la première extrémité dudit tube étant fermée par la surface d'une membrane filtrante (102) immobilisée par collage sur la section des parois dudit tube,
- un piston (104) comprenant une tige (107) reliée à un moyen d'appui (108), ladite tige coulissant selon un axe parallèle aux parois du tube (101), ledit piston se déplaçant librement selon l'axe vertical du tube (101), et les bords du moyen d'appui (108) du piston (104) n'étant pas en contact permanent avec la surface de la paroi interne du tube (101),
- un bloc (103) de matériau absorbant hydrophile placé à l'intérieur du tube (101), intercalé entre (i) la surface interne de la membrane filtrante (102) et (ii) le moyen d'appui (108) du piston (104), ledit matériau absorbant hydrophile ayant la propriété de gonfler lorsqu'il est mis en contact avec un milieu liquide aqueux.

2. Dispositif selon la revendication 1, **caractérisé en ce que** le bloc (103) consiste en bloc de viscose compressé.

3. Dispositif selon l'une des revendications 1 à 2, **caractérisé en ce que** la membrane filtrante possède une taille de pore allant de 1 µm à 25 µm.

4. Dispositif selon l'une des revendications 1 à 3, **caractérisé en ce que** la seconde extrémité dudit tube est fermée par un bouchon (105) comprenant un orifice central (106).

5. Dispositif selon l'une des revendications 1 à 4, **caractérisé en ce qu'**un disque (110) est fixé sur la tige (107), le diamètre dudit disque (110) étant déterminé de manière à permettre le coulissement de la tige (107) selon un axe parallèle aux parois du tube (101).

6. Dispositif selon l'une des revendications 1 à 4, **caractérisé en ce que** ledit tube (101) comprend deux sections formant une surface externe continue, respectivement :
- une première section S1 de type cylindrique dont une extrémité consiste en la première extrémité dudit tube qui est fermée par la surface d'une membrane filtrante (102), et dont l'autre extrémité forme une surface externe continue avec la deuxième section, et
- une deuxième section S2 de type tronconique, dont l'extrémité de plus petit diamètre forme une surface externe continue avec ladite première section cylindrique, et dont l'extrémité de plus grand diamètre consiste en la seconde extrémité du tube (101).

7. Dispositif selon la revendication 6, **caractérisé en ce que** l'extrémité de plus grand diamètre de la section S2 du tube (101) comprend un épaulement (111) annulaire plat perpendiculaire aux bords de la section S1 du tube (101).

8. Procédé adapté à la capture des particules biologiques en suspension dans un milieu liquide comprenant les étapes suivantes :
a) placer un dispositif selon l'une des revendications 1 à 7 dans un récipient contenant un milieu liquide dans lequel des particules biologiques sont en suspension,
b) maintenir le dispositif dans ledit récipient pendant une durée suffisante pour capturer au moins une partie des particules biologiques contenues dans le milieu liquide sur la surface externe de la membrane filtrante (102) du dispositif (101),

9. Procédé selon la revendication 8, lequel est en outre adapté à la réalisation d'une préparation cytologique à partir d'un milieu liquide contenant des particules biologiques en suspension, comprenant les étapes additionnelles suivantes :
c) retirer le dispositif dudit récipient, et
d) récupérer au moins une partie des particules biologiques retenues sur la membrane filtrante du dispositif

10. Procédé selon la revendication 9, caractérisé en que, à l'étape d), une pression est exercée sur le bloc (103) par action sur le piston (104), de manière à générer un flux de liquide allant de l'intérieur du dispositif (101) vers l'extérieur, ledit flux de liquide provoquant le détachement des particules biologiques initialement retenues sur la membrane filtrante.

11. Procédé selon l'une des revendications 9 et 10, **caractérisé en ce qu'**à l'étape d), on transfère au moins une partie des particules biologiques de la membrane filtrante du dispositif vers la surface d'un support d'analyse cytologique, par mise en contact de ladite membrane filtrante avec la surface dudit support d'analyse cytologique.

12. Procédé selon la revendication 11, **caractérisé en ce que** le procédé comprend l'étape additionnelle suivante :
e) réaliser une coloration des particules biologiques transférées sur la surface dudit support d'analyse cytologique.

13. Procédé selon l'une des revendications 9 et 10, **caractérisé en ce qu'**à l'étape d), on transfère au moins une partie des particules de la membrane filtrante du dispositif vers un conteneur, pour obtenir une préparation cytologique sous la forme d'une suspension concentrée de cellules.

14. Procédé selon la revendication 9, **caractérisé en ce qu'**à l'étape d), on récupère au moins une partie des particules biologiques retenues sur la membrane filtrante par grattage de ladite membrane filtrante.

15. Plateforme multi-essais comprenant une pluralité de dispositifs pour capturer des particules biologiques tels que définis dans l'une des revendications 1 à 7.

16. Système adapté à la capture des particules biologiques en suspension dans un milieu liquide, ledit système comprenant la combinaison de deux éléments :
- un premier élément qui consiste en une plateforme multi-essais selon la revendication 15, qui comprend une pluralité de dispositifs pour capturer des particules biologiques disposés, dans ladite plateforme, selon un arrangement déterminé, et
- un portoir destiné à recevoir des récipients pour échantillons biologiques, lesdits récipients pouvant être disposés, dans ledit portoir, selon un arrangement compatible avec l'arrangement des dispositifs dans ladite plateforme multi-essais.

17. Procédé adapté à la réalisation d'une préparation cytologique à partir d'un milieu liquide contenant des particules biologiques en suspension, comprenant les étapes suivantes :
a) placer au moins une partie de la pluralité de dispositifs pour capturer des particules biologiques contenus dans une plateforme multi-essais telle que définie dans la revendication 15 dans un récipient, ou une pluralité de récipients, contenant chacun un milieu liquide dans lequel des particules biologiques sont en suspension,
b) maintenir le ou les dispositif(s) dans ledit ou lesdits récipient(s) pendant une durée suffisante pour capturer au moins une partie des particules biologiques contenues dans le milieu liquide sur la surface externe de la membrane filtrante (102) de chacun des dispositifs (101),
c) réaliser une translation de la plateforme multi-essais afin de retirer le ou les dispositif(s) dudit récipient ou desdits récipients correspondant(s), et d) récupérer au moins une partie des particules biologiques retenues sur la membrane filtrante de chacun des dispositifs contenus dans la plateforme multi-essais ayant été placés dans un récipient.

## Patentansprüche

1. Vorrichtung zum Fangen von biologischen Partikeln, die in einem flüssigen Medium suspergiert sind, die dazu ausgelegt ist, biologische Proben zur zytologischen Analyse bereitzustellen, umfassend:
- ein Rohr (101) mit einem ersten und einem zweiten Ende,
- wobei das erste Ende des Rohrs von der Oberfläche einer Filtermembran (102) verschlossen ist, die durch Kleben an einem Abschnitt der Wände des Rohrs befestigt ist,
- ein Kolben (104), der eine Stange (107) umfasst, die mit einem Abstützmittel (108) verbunden ist, wobei die Stange entlang einer parallel zu den Wände des Rohrs (101) verlaufenden Achse verschiebbar ist und die Ränder des Abstützmittels (108) des Kolbens (104) nicht in dauerhaftem Kontakt mit der Innenwandoberfläche des Rohrs (101) stehen,
- einen Block (103) aus hydrophilem, absorbierendem Material, der im Innern des Rohrs (101) angeordnet ist und eingeschlossen ist zwischen (i) der Innenfläche der Filtermembran (102) und (ii) dem Abstützmittel (108) des Kolbens (104), wobei das hydrophile, absorbierende Material die Eigenschaft hat, aufzuquellen, wenn es in Kontakt mit einer wässrigen Flüssigkeit gebracht wird.

2. Vorrichtung nach einem der Ansprüche 1 und 2, **dadurch gekennzeichnet, dass** der Block (103) aus komprimierte Viskose-Block besteht.

3. Vorrichtung nach einem der Ansprüche 1 bis 2, **dadurch gekennzeichnet, dass** die Filtermembran eine Porengröße im Bereich von 1 um bis 25 um hat.

4. Vorrichtung nach einem der Ansprüche 1 bis 3, **dadurch gekennzeichnet, dass** das zweite Ende des Rohres durch einen Stopfen verschlossen ist (105), der eine zentrale Öffnung (106) hat.

5. Vorrichtung nach einem der Ansprüche 1 bis 4, **dadurch gekennzeichnet, dass** eine Scheibe (110) auf der Stange (107) befestigt ist, wobei der Durchmesser der Scheibe (110) so bestimmt wird, dass das Gleiten der Stange (107) entlang einer Achse ermöglicht wird, die parallel zu den Wände des Rohrs (101) verläuft.

6. Vorrichtung nach einem der Ansprüche 1 bis 4, **dadurch gekennzeichnet, dass** das Rohr (101) zwei Abschnitte hat, die jeweils eine kontinuierliche Außenfläche bilden:
- einen ersten Abschnitt S1 von zylindrischem Typ, dessen eines Ende das erste Ende des Rohrs, das durch die Oberfläche einer Filtermembran (102) verschlossen ist, und dessen anderes Ende eine stetige Außenfläche mit dem zweiten Abschnitt bilden, und
- einen zweiten Abschnitt S2 vom kegelstumpfartigen Typ, wobei das Ende mit dem kleineren Durchmesser eine stetige Außenfläche mit dem ersten zylindrischen Abschnitt bildet und das Ende mit dem größeren Durchmesser aus dem zweiten Ende des Rohrs (101) besteht.

7. Vorrichtung nach Anspruch 6, **dadurch gekennzeichnet, dass** das Ende des Abschnitts S2 des Rohres (101) mit dem größeren Durchmesser einen flachen, ringförmigen und senkrecht zu den Rändern des Abschnitts S1 des Rohrs (101) verlaufenden Absatz (111) hat.

8. Verfahren zum Fangen von biologischen Partikeln, die in einem flüssigen Medium suspergiert sind, umfassend die Schritte:
a) Anordnen einer Vorrichtung nach einem der Ansprüche 1 bis 7 in einem Behälter, der ein flüssiges Medium enthält, in dem biologische Partikel suspergiert sind,
b) Halten der Vorrichtung in dem Behälter für eine ausreichende Zeit, um zumindest einen Teil der biologischen Partikel, die in dem flüssigen Medium enthalten sind, auf der Außenfläche der Filtermembran (102) der Vorrichtung (101) zu fangen.

9. Verfahren nach Anspruch 8, das ferner zur Herstellung eines zytologischen Präparats aus einem flüssigen Medium ausgelegt ist, im welchem biologischen Partikel in Suspension enthalten sind, umfassend die folgenden Schritte:
c) Herausziehen der Vorrichtung aus dem Behälter, und
d) Gewinnung mindestens eines Teils der biologischen Partikel, die auf der Filtermembran der Vorrichtung gefangen sind.

10. Verfahren nach Anspruch 9, **dadurch gekennzeichnet, dass** in Schritt d) durch die Wirkung des Kolbens (104) ein Druck auf den Block (103) ausgeübt wird, um so eine Strömung der Flüssigkeit zu erzeugen, die aus dem Inneren der Vorrichtung (101) nach außen verläuft, wobei der Flüssigkeitsstrom die Ablösung der biologischen Partikel bewirkt, die zunächst auf der Filtermembran gefangen sind.

11. Verfahren nach einem der Ansprüche 9 und 10, **dadurch gekennzeichnet, dass** in Schritt d) zumindest einen Teil der biologischen Partikel von der Filtermembran der Vorrichtung auf die Oberfläche eines zytologischen Analyseträgers übertragen werden, indem die Filtermembran der mit der Oberfläche des zytologischen Analyseträgers in Kontakt gebracht wird.

12. Verfahren nach Anspruch 11, **dadurch gekennzeichnet, dass** das Verfahren den folgenden zusätzlichen Schritt umfasst:
e) Färben der biologischen Partikel, die auf die Oberfläche des zytologischen Analyseträgers übertragen wurden.

13. Verfahren nach einem der Ansprüche 9 und 10, **dadurch gekennzeichnet, dass** in Schritt d) weinigstens einige der Teilchen der Filtermembran der Vorrichtung in einen Behälter übertragen werden, um ein zytologisches Präparat in Form einer konzentrierten Zellsuspension zu gewinnen.

14. Verfahren nach Anspruch 9, **dadurch gekennzeichnet, dass** in Schritt d) zumindest ein Teil der biologischen Teilchen auf der Filtermembran durch Abschaben der Filtermembran gewonnen werden.

15. Multi-Assay-Plattform für die Erfassung biologischer Teilchen, die eine Vielzahl von Vorrichtungen nach einem der Ansprüche 1 bis 7 umfasst.

16. System zum Fangen von in einem flüssigen Medium suspergierten biologische Partikeln, wobei das System die Kombination aus zwei Elementen umfasst:
- als erstes Element eine Multi-Assay-Plattform gemäß Anspruch 15, die eine Vielzahl von Vorrichtungen zur Erfassung von biologischen Partikeln umfasst, die in einer bestimmten Anordnung auf der Plattform angeordnet sind, und
- einen Träger zur Aufnahme von Behältern für biologische Proben, wobei die Behälter in dem Gestell gemäß einer Anordnung positioniert sind, die kompatibel mit der Anordnung der Vorrichtungen in der Multi-Assay-Plattform ist.

17. Verfahren zur Herstellung eines zytologischen Präparats aus einem flüssigen Medium, welches biologische Partikel in Suspension enthält, umfassend die folgenden Schritte:
a) Anordnen zumindest eines Teils der Mehrzahl von Vorrichtungen zum Fangen von biologischen Partikeln, die in Multi-Assay-Plattform nach Anspruch 15 enthalten sind, in einem Behälter oder eine Mehrzahl von Behältern, die jeweils ein flüssiges Medium enthalten, in dem biologische Partikel suspergiert sind,
b) Halten oder Vorrichtung(en) in dem einen oder den mehreren Behälter(n) für eine ausreichende Zeit, um zumindest einen Teil der biologischen Partikel in dem flüssigen Medium auf der Außenfläche der Filtermembran (102) jeweiligen Vorrichtungen (101) zu adsorbieren,
c) Bewegen der der Multi-Assay-Plattform, um die eine oder die mehreren Vorrichtung(en) aus dem entsprechenden Behälter oder den Behälter(n) herauszuziehen, und
d) Gewinnen zumindest eines Teils der biologischen Partikel, die auf der Filtermembran der jeweiligen Vorrichtungen der Multi-Assay-Plattform adsorbiert sind, die in dem Behälter angeordnet worden waren.

## Claims

1. A device for capturing suspended biological particles in a liquid medium and for preparing biological samples for cytological analysis, comprising:
- a tube (101) comprising first and second ends,
- the first end of said tube being closed by the surface of a filter membrane (102) rendered stationary by adhesion onto the cross-section of the walls of said tube,
- a piston (104) comprising a rod (107) connected to a bearing means (108), said rod sliding along an axis parallel to the walls of the tube (101), the said piston moving freely along the vertical axis of the tube (101), and the edges of the piston (104) bearing means (108) not being continuously contacting the surface of the inner wall of the tube (101),
- a block (103) of hydrophilic absorbent material placed inside the tube (101), inserted between (i) the inner surface of the filter membrane (102) and (ii) the piston (104) bearing means (108), the said absorbent material being capable to swell when contacting an aqueous liquid medium.

2. A device according to claim 1, **characterized in that** the block (103) consists in a block of compressed viscose.

3. A device according to any of claim 1 or 2, **characterized in that** the filter membrane has a pore size ranging from 1 µm to 25 µm.

4. A device according to any of claim 1 to 3, **characterized in that** the second end of said tube is closed by a plug (105) comprising a central hole (106).

5. A device according to any of claim 1 to 4, **characterized in that** a disk (110) is fixed on the rod (107), the diameter of said disk (110) being determined so as to allow the sliding of the rod (107) along an axis parallel to the walls of the tube (101).

6. A device according to any of claim 1 to 4, **characterized in that** said tube (101) comprises two sections forming a continuous outer surface, respectively:
- a first section S1 of the cylindrical type, one end of which consists in the first end of said tube which is closed by the surface of a filter membrane (102), and which other end forms a continuous outer surface with the second section, and
- a second section S2 of the tapered type, which end with the smallest diameter forms a continuous outer surface with said first cylindrical section, and which end with the largest diameter consists in the second end of the tube (101).

7. A device according to claim 6, **characterized in that** the end with the largest diameter of the section S2 of the tube (101) comprises a flat, annular shoulder (111) perpendicularly to the edges of the section S1 of the tube (101).

8. A method for capturing suspended biological particles in a liquid medium, including the following steps of:
a) placing a device according to any of claim 1 to 7 in a container containing a liquid medium with biological particles suspended therein,
b) maintaining the device in said container for a time sufficient for capturing at least part of the biological particles contained in the liquid medium on the outer surface of the filter membrane (102) of the device (101).

9. A method according to claim 8, which is further for making a cytological preparation from a liquid medium containing suspended biological particles, comprising the additional steps of:
c) removing the device from said container, and
d) recovering at least part of the biological particles retained on the filter membrane of the device.

10. A method according to claim 9, **characterized in that**, in step d), a pressure is exerted onto the block (103) by actuating the piston (104), so as to generate a liquid flow from the inside of the device (101) to the outside, said liquid flow causing the detachment of the biological particles initially retained on the filter membrane.

11. A method according to any of claim 9 and 10, **characterized in that** in step d), at least part of the biological particles is transferred from the filter membrane of the device onto the surface of a cytological analysis support, by contacting said filter membrane with the surface of said cytological analysis support.

12. A method according to claim 11, **characterized in that** the method comprises the following additional step:
e) performing the staining of the bioparticles transferred onto the surface of said cytological analysis support.

13. A method according to any of claim 9 and 10, **characterized in that** in step d), at least part of the biological particles are transferred from the filter membrane of the device to a container, for obtaining a cytological preparation in the form of a cell concentrated suspension.

14. A method according to claim 9, **characterized in that** in step d), at least part of the biological particles retained on the filter membrane is recovered by scraping said filter membrane.

15. A multi-assay platform comprising a plurality of biological particle capture devices according to any of claim 1 to 7.

16. A system for capturing suspended bioparticles in a liquid medium, said system comprising the combination of two elements:
- a first element consisting in a multi-assay platform according to claim 15, which includes a plurality of bioparticle capture devices positioned in said platform according to a determined arrangement, and
- a sample rack to receive the biological sample containers, where said containers may be positioned in said sample rack according to an arrangement compatible with the arrangement of the devices in said multi-assay platform.

17. A method for making a cytological preparation from a liquid medium containing suspended biological particles, comprising the following steps of:
a) placing at least part of the plurality of bioparticle capture devices contained in a multi-assay platform such as defined in claim 15 in a container, or a plurality of containers, containing each a liquid medium with biological particles suspended therein,
b) maintaining the device(s) in said container(s) for a time sufficient for capturing at least part of the biological particles contained in the liquid medium on the outer surface of the filter membrane (102) of each of the devices (101),
c) performing a multi-assay platform translation so as to remove the device(s) from said corresponding container(s), and
d) recovering at least part of the biological particles retained on the filter membrane of each of the devices contained in the multi-assay platform having been placed in a container.
